# EUROPEAN PATENT SPECIFICATION

(11) **EP 3 300 479 B1**
(45) Date of publication and mention of the grant of the patent: **09.12.2020**
(21) Application number: 16730645.5
(22) Date of filing: 07.06.2016
(51) Int. Cl.: A61B 17/74

(54) **AN INTRAMEDULLARY FIXATION APPARATUS AND SYSTEM FOR USE IN HIP AND FEMUR FRACTURE SURGERY**
MARKFIXIERVORRICHTUNG UND SYSTEM ZUR VERWENDUNG IN DER HÜFT- UND FEMURFRAKTURCHIRURGIE
SYSTÈME ET APPAREIL DE FIXATION INTRAMÉDULLAIRE DESTINÉS À ÊTRE UTILISÉS DANS UNE CHIRURGIE DE FRACTURE DE LA HANCHE ET DU FÉMUR

(30) Priority: 09.06.2015 US 201514735068
(43) Date of publication of application: 04.04.2018
(73) Proprietor: Savage Medical Design LLC, Potsdam, NY 13676 (US)
(72) Inventor: SAVAGE, John B., Potsdam, New York 13676-1164 (US)
(74) Representative: Mummery, Thomas Zack
(86) International application number: PCT/US2016/036267
(87) International publication number: WO 2016/200839

(56) References cited:
- EP-A1- 2 730 243
- WO-A1-2012/099944
- WO-A2-02/098330
- US-A1- 2008 140 077
- US-A1- 2014 243 826

## Description

### 1. Field of Technology.

At least some embodiments disclosed herein relate, in general, to the field of orthopaedic implant apparatus and systems for bone and joint surgery, and more specifically, to intramedullary fixation apparatus and systems for certain types of fractures, including, but not limited to, fractures of the hip and femur.

### 2. Background.

An open reduction and internal fixation (ORIF) is a type of orthopaedic surgery used to repair fractured bones. This is a two-part surgery, First, the broken bone is *reduced* or put back into place. Next, an *internal fixation* device is placed on or in the bone, or both, typically through the use of screws, plates, rods, pins or nails used to hold the broken bone together. The Dynamic Hip Screw and the Gamma Nail are currently two acceptable fixation apparatus to treat unstable intertrochanteric and sub-trochanteric fractures, which are common in the old osteoporotic patient but can be challenging to fix and problematic to manage.

In the 1980s, perhaps the most common method of fixation employed the Dynamic Hip Screw. Typically, there are three (3) components of a Dynamic Hip Screw, including a dynamic lag screw (inserted into the neck of a femur), a side plate, and a plurality of cortical screws (fixated to proximal or distal femoral shaft, or both). The idea behind this design is that the femoral head component is allowed to move along one plane - a Dynamic Hip Screw allows controlled dynamic sliding of the femoral head component along the construct --- and since bone responds to dynamic stresses, the native femur may undergo remodeling and proper fracture healing through compression of the fracture line. A disadvantage of this technique, however, was that the plate was lateral to the load-bearing line of the hip, such that any defect in the medial cortex of the femur, whether due to imperfect reduction, comminution, or a metastasis meant that a varus stress would be applied to the fixation with every weight- bearing step, which could, in turn, cause the cutting-out of the screw from the head of the femur, or failure at the nail-plate junction or of the screws securing the plate to the bone.

An intramedullary appliance, the Zickel Nail, addressed some of these problems, but it proved technically difficult to insert, even in experienced hands, and presented its own problems. Among these was the increased likelihood of fracture at the base of the greater trochanter.

The Gamma Nail is also of an intramedullary fixation design, developed for semi-closed insertion. A Gamma Nail has three principle components: an intramedullary rod (nail) passed down the medullary cavity of the upper shaft of the femur, a lag screw passed through a hole in the proximal part of the rod and from there inserted into the head of the femur, and a set screw which prevents rotation of the main screw. The Gamma Nail itself can be somewhat difficult to place, and biomechanical experiments have suggested that while the sliding ability of the lag screw is maintained in the Gamma Nail, it is decreased in comparison with that of the Dynamic Hip Screw. This may be a particular problem in heavy set or obese patients, for whom it may be difficult to obtain access to the trochanter and find an optimal access point for introduction of a lag screw. Moreover, accidental fracture and over-penetration by the lag screw are not uncommon with the Gamma Nail design, and an optimal level of compression of the fractured bone may not always be obtained during surgery.

EP 2730243 discloses a device having a cephalic screw (2) inserted partly in head and neck of femur and comprising a transverse opening to receive a femoral saber (1). The opening has a distal transverse wall (252) and a proximal transverse wall (251) tilted in same direction. Distance between an upper edge of the distal transverse wall located in a plane of inlet orifice of the transverse opening and an orthogonal projection of lower edge of the proximal transverse wall in a same plane of the inlet orifice is greater than or equal to width of the saber.

WO02098330 discloses a device for the proximal locking of an intramedullary nail for osteosynthesis of fractures in the trochanteric region of the femur, said nail being provided with a transverse through-hole, characterized in that it comprises a transcervical longitudinal element self-threading at one end for fixing to the head of the femur and provided at the other end with an opening for receiving an insertion instrument which performs a translational movement thereof within a bell which can be stably positioned inside said transverse hole of said nail, during said translation a rotation of said transcervical longitudinal element inside said bell being prevented, said translation being able to occur between two opposite stop positions, at least one of which is determined by the bearing contact of a pin which extends integrally from said bell into a slot formed in the surface of said transcervical longitudinal element.

### DESCRIPTION

The invention is as defined in the claims.

### BRIEF DESCRIPTION OF THE SEVERAL VIEWS OF THE DRAWINGS

The embodiments illustrated are by way of example, and not limitation, in the figures of the accompanying drawings in which like references indicate similar elements.
**FIG. 1** is an isometric view of an intramedullary nail, a dynamic lag screw structure comprised of a longitudinal sheath and a lag screw, and a surgical screw as a fixation means.
**FIG. 2** is cutaway view of a dynamic lag screw structure with a lag screw inserted fully into a longitudinal sheath, revealing a dynamic channel for introduction of an intramedullary nail through the dynamic lag screw structure.
**FIG. 3** is an isometric view of a femur, the head and neck of which have been reamed to accept a dynamic lag screw structure, with the longitudinal sheath thereof in position for insertion.
**FIG. 4** is an isometric view of a femur into which the longitudinal sheath of a dynamic lag screw structure has been inserted, and the lag screw of the dynamic lag screw structure is in position for insertion within the sheath and thence into the neck and head of the femur.
**FIG. 5** is an isometric view of a femur, reamed for placement of an intramedullary nail through a dynamic lag screw structure via a dynamic channel created by the alignment of an upper orifice and a lower orifice of a longitudinal sheath with a slot in a lag screw.
**FIG. 6** is an isometric view of a femur into which an intramedullary nail has been inserted through the dynamic lag screw structure, and of a surgical screw in position to be installed distally as a fixation means to secure the intramedullary nail in position with respect to the femur.
**FIG. 7** is an isometric view of a femur into which an intramedullary nail has been inserted through the dynamic lag screw structure, and into which a surgical screw has been inserted distally through a lateral orifice in the intramedullary nail.
**FIG. 8** is a cross-sectional view of an intramedullary nail that has been inserted into the medullary shaft of a femur through the dynamic lag screw structure, revealing an external lip of a lag screw engaged with the inner lip of a longitudinal sheath, and the external rim of a longitudinal sheath engaged against the lateral cortex of the femur.

### DETAILED DESCRIPTION OF THE INVENTION

The following description and drawings are illustrative and are not to be construed as limiting. Numerous specific details are described to provide a thorough understanding. However, in certain instances, well-known or conventional details are not described in order to avoid obscuring the description. References to "one embodiment" or "an embodiment" in the present disclosure are not necessarily references to the same embodiment; and, such references mean at least one.

Reference in this specification to "one embodiment" or "an embodiment" means that a particular feature, structure, or characteristic described in connection with the embodiment is included in at least one embodiment of the disclosure. The appearances of the phrase "in one embodiment" or substantially similar phrases in various places in the specification are not necessarily all referring to the same embodiment, nor are separate or alten1ative embodiments mutually exclusive of other embodiments. Moreover, various features are described which may be exhibited by some embodiments and not by others. Similarly, various requirements are described which may be requirements for some embodiments but not other em bodiments.

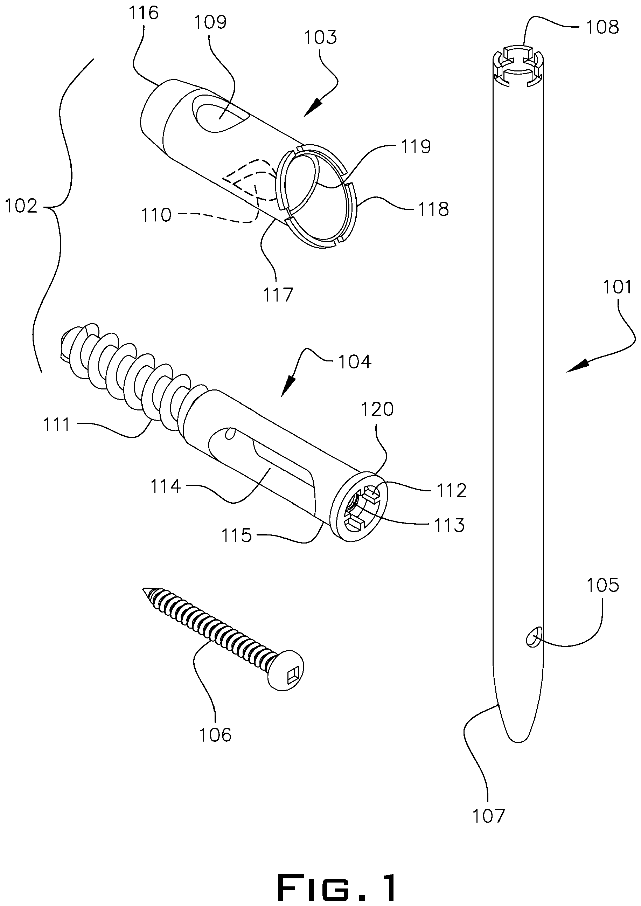

As illustrated in **FIG. 1**, an embodiment of the system comprises an intramedullary nail (intramedullary rod) **101**, a dynamic lag screw structure **102** comprising a longitudinal sheath **103** and a lag screw **104**, and some securing means, such as surgical screws, bolts, or tightrope fixation, now known or later to be developed in the art, to keep an intramedullary nail **101** in a proper position relative to a bone. By way of illustration and without limitation, in an embodiment, an intramedullary nail may have one or more lateral orifices **105** passing through it proximally or distally, or both, with respect to a long bone, each such lateral orifice **105** capable or accepting one or more surgical screws **106** designed to pass into or through an intramedullary nail **101.** As is known in the art, an intramedullary nail **101** may have a solid, semisolid, or hollow core, may be curved to accommodate the anterior curvature of a femur of a patient, and may be of varying length, as well as and proximal and distal diameter, as may be appropriate to a given patient. An intramedullary nail **101** may be tapered at its distal end **107**, *inter alia,* to facilitate insertion, and may be configured at its proximal end **108** to receive a guidance device or comparable instrument currently known or later to be developed in the art.

In an embodiment, a longitudinal sheath **103** of a dynamic lag screw structure **102** comprises an upper orifice **109** and lower orifice **110**, together capable of accepting an intramedullary nail **101** and allowing said intramedullary nail **101** to be passed through, and perpendicular to, said longitudinal sheath **103.** In an embodiment, the distal end (insertion end) **116** of a longitudinal sheath **103** may be tapered, and at its proximal end **117** may have an outer rim **118** and an inner lip **119.** An outer rim **118** may ensure that a longitudinal sheath **103** cannot be inserted beyond the lateral cortex of a femur. In an embodiment, a longitudinal sheath **103** may be pressed or tapped into the bone through a hole or channel that has been reamed in a bone.

In an embodiment, a lag screw **104** may employ at its distal end (insertion end) a self-tapping thread **111.** In an embodiment, a lag screw **104** may have at its proximal end a drive **112.** Said drive **112** may include some form of internal threading **113** or other securing means capable of receiving a self-holding screwdriver or comparable tool now known or later to be developed in the art. In an embodiment, a lag screw **104** may have longitudinal slot **114** through its shank **115** capable of accepting an intramedullary nail **101** and allowing it to pass through said lag screw **104.** In an embodiment, a lag **screw104** may have an external lip **120** at its proximal end capable of engaging an inner lip **117** of a longitudinal sheath **103** so as to prevent said lag screw **104** from further penetrating the bone.

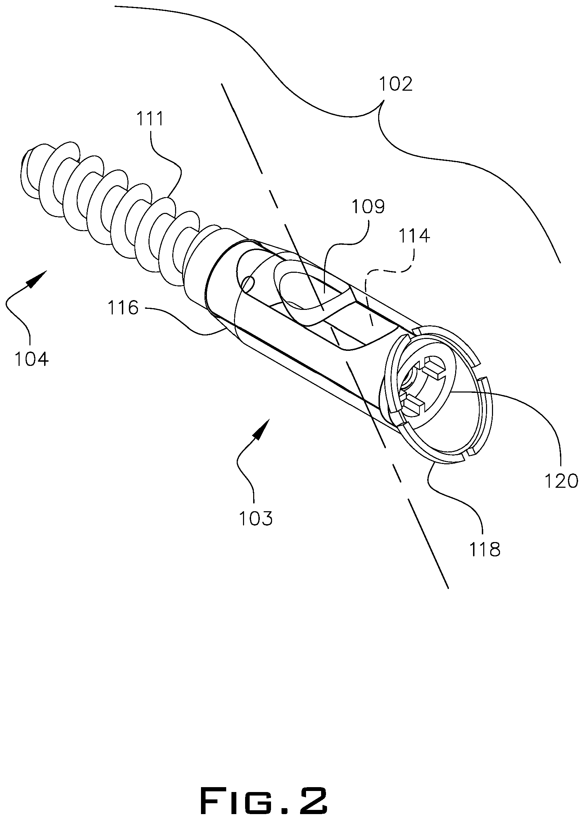

As illustrated in **FIG. 2**, in an embodiment, a lag screw **104** may be inserted into a longitudinal sheath **103**, and an intramedullary nail **101** passed through a dynamic channel formed by the alignment of both an upper orifice **109** and a lower orifice **110** of said longitudinal sheath **103** with a longitudinal slot **114** in the shank **115** of an inserted lag screw **104**, and hence, into the medullary shaft of a femur. Such a dynamic lag screw structure **102** or comparable structure may provide a means of allowing the dynamic adjustment of the position of the lag screw **104** in three dimensions - longitudinally, laterally, and normally (i.e., vertically)-relative to the intramedullary nail **101**, and hence, to the shaft of the femur and to the neck and head of the femur, respectively, permitting further compression of the fractured bone as a patient begin s to bear weight on the joint following surgery.

An embodiment may be comprised of various types of stainless steel, titanium, titanium alloys, biodegradables and other suitable materials, alone or in combination, currently known in the art or which may later be developed in the art or for use in the art

An embodiment may have a distal diameter for an intramedullary nail **101** within an approximate range of 11 mm to 15 mm, with a proximal diameter within an approximate range of 15 mm to 16 mm, and a length within an approximate range of 250 mm to 500 mm. In an embodiment, a lag screw **103** may have a diameter within an approximate range of 10 mm to 11 mm, a core diameter within an approximate range of 6 mm to 7 mm, and a length within an approximate range of 50 to 145 mm. An embodiment may utilize a surgical screw **106** that is either full or partially threaded and self-tapping, with a diameter within an approximate range of 4 mm to 6 mm, and a length within an approximate range of 15 mm to 130 mm.

An embodiment of the current invention allows it to be placed in the hip simply and quickly using an external guidance device or system. Such an initial placement may be thought of as similar to the placement of a screw for in-situ pinning.

After reduction of a fracture of a proximal femur of a patient on a fracture table, if needed, and positioning of a fluoroscope or similar guidance device or system over the fractured proximal femur, a small incision (typically less than an inch) may be made laterally to the fractured proximal femur and an intramedullary nail 101, which could be a Steinman Pin, may be inserted into the neck and head of the femur, with or without reaming, to a depth appropriate to the anatomy of the patient, centrally and slightly posterior in the head of the fractured proximal femur. An intramedullary nail **101** may then be placed with the assistance of a guidance device or system to assure a proper angle of the intramedullary nail **101** in relation to the shaft of the fractured proximal femur. A desired depth into which a lag screw **104** is to be inserted into the neck and head of a femur may then be determined.

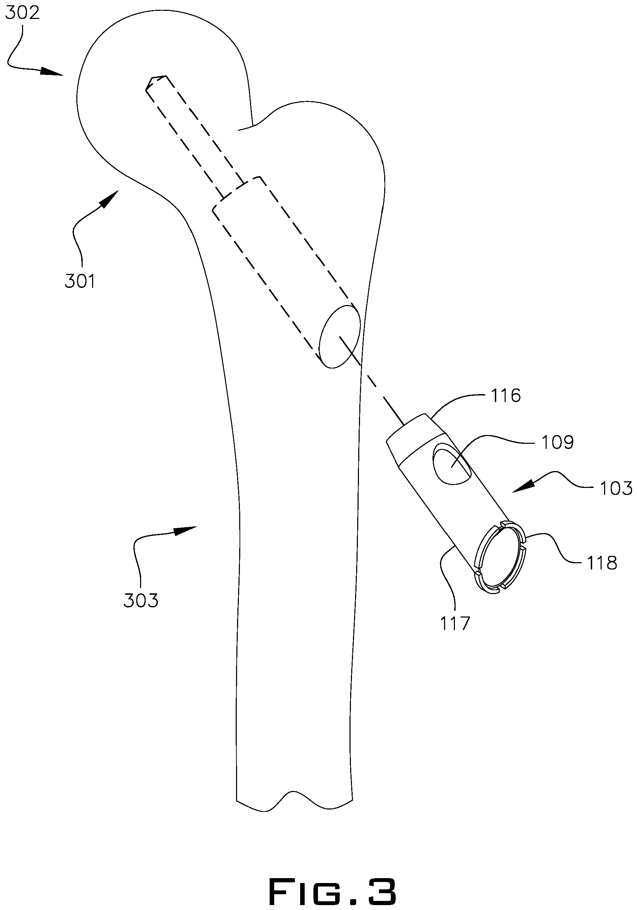

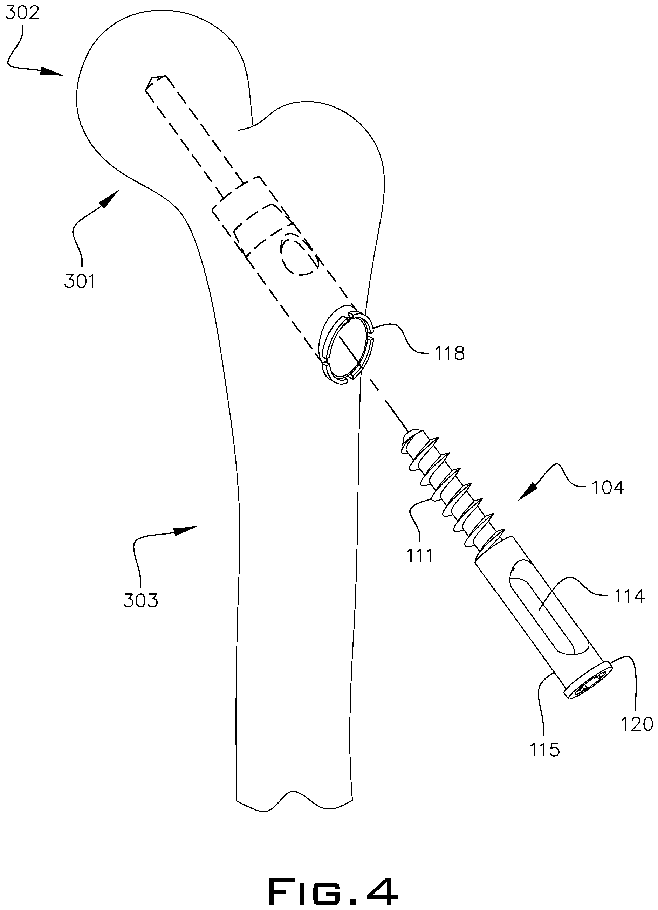

As illustrated in **FIG.** 3 and **FIG. 4**, the neck **201** and head **202** of a femur **203** then may be reamed in a two-step process. First, a channel may reamed at a desired angle from the lateral cortex of the fractured proximal femur to the center of the head of the fractured proximal femur at the desired depth for the lag screw previously determined. Second, a wider and shorter channel may be reamed at approximately the same angle to accommodate a longitudinal sheath **103.** Next, a longitudinal sheath **103** may be pressed or tapped into the bone until any external rim of the sheath reaches the lateral cortex of the fractured proximal femur.

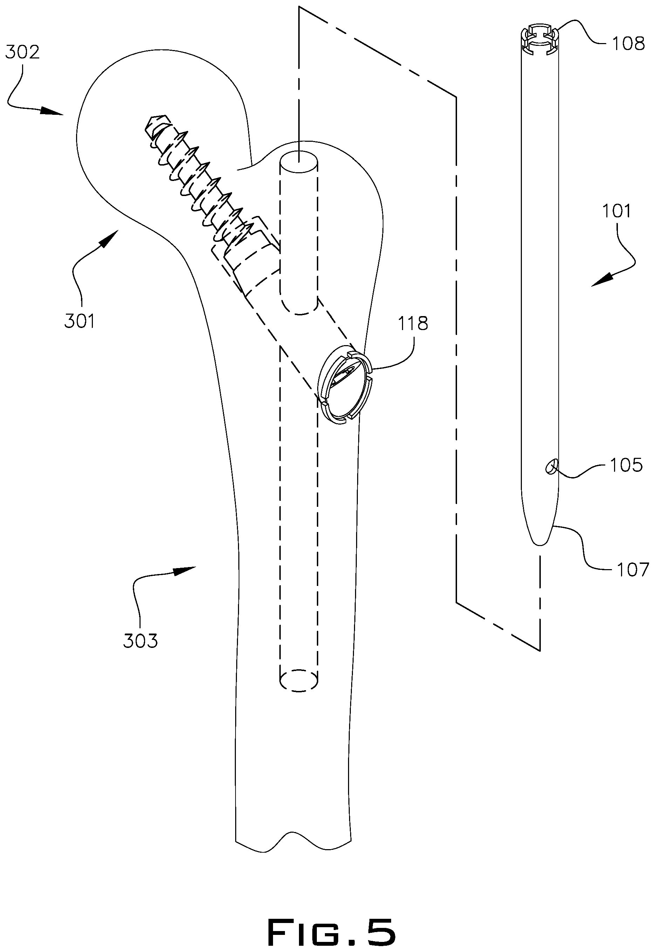

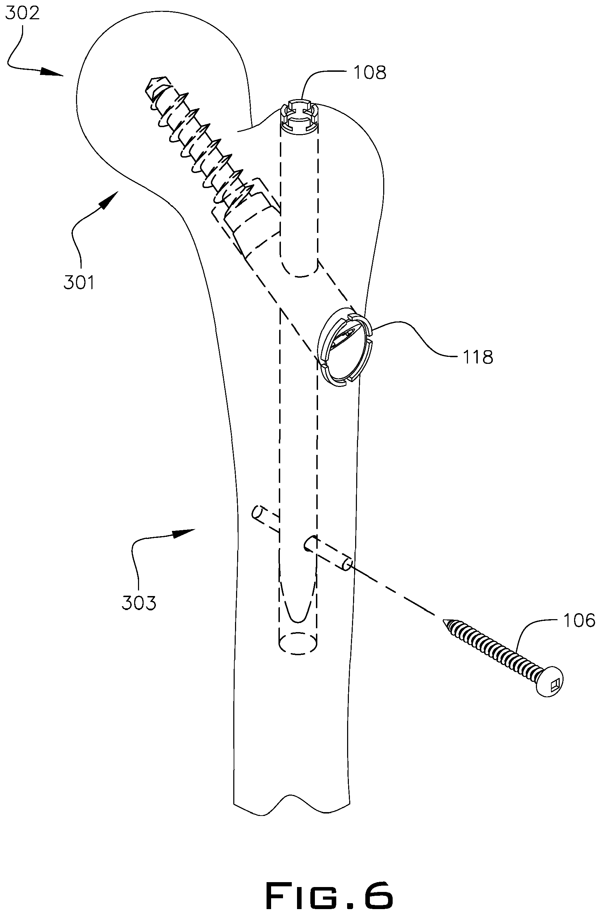

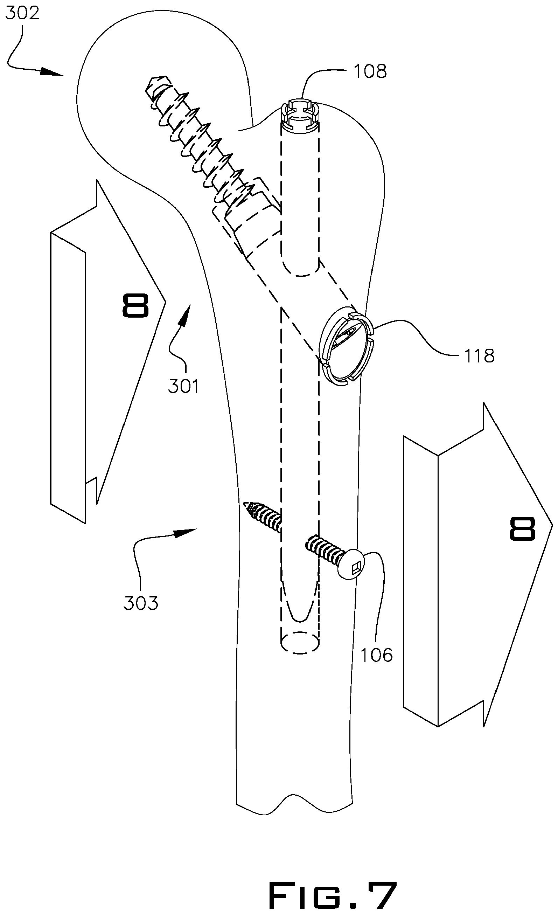

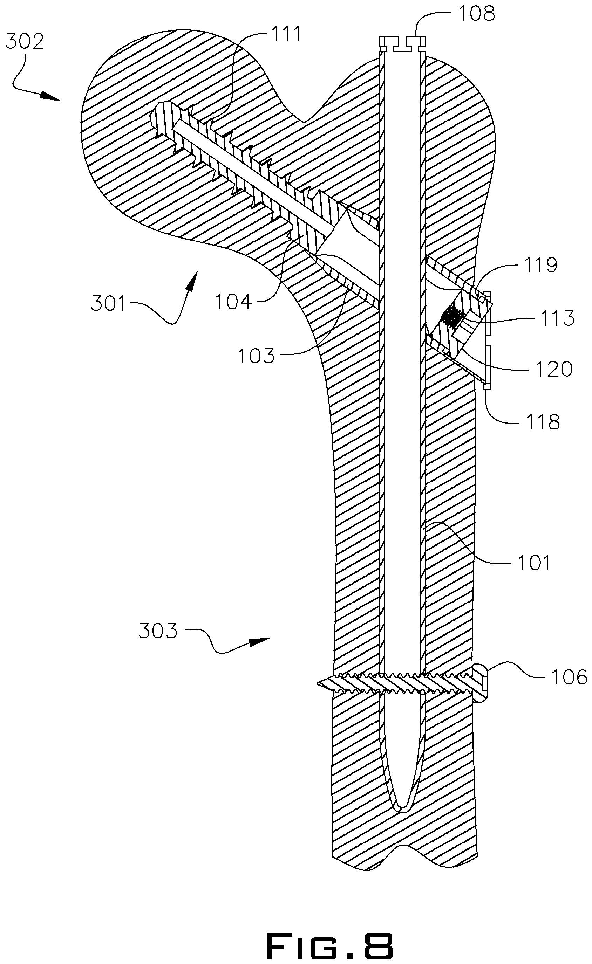

As illustrated in **FIG. 4** and **Fig. 5**, a lag screw **104**, which may have been attached to a self-holding screwdriver or comparable device, may then be inserted into and through the longitudinal sheath **103** embedded in the bone and screwed or otherwise inserted into the neck and head of a femur and into the longer, narrower channel initially reamed. As illustrated in **FIG. 6**, an upper orifice **109** and a lower orifice **110** in a longitudinal sheath **103** and a slot **114** in a lag screw **104** which has been inserted into a longitudinal sheath **103** may aligned, and the resulting dynamic channel for introduction of an intramedullary nail **101** lined up with the medullary canal of a femoral shaft. A fluoroscope or other guidance device or system then may be used to place an intramedullary nail **101** through the trochanter into the construct and canal of a femur. As depicted in **FIG. 6**, **FIG. 7** and **FIG. 8**, an intramedullary nail **101** then may be secured to the bone with one or more surgical screws **106** utilizing a fluoroscope or other guidance device or system.

## Claims

1. An orthopaedic fixation apparatus and system comprising:
a dynamic lag screw structure (102) comprising
a lag screw (104), the lag screw comprising a longitudinal slot (114) through a shank (115) of the lag screw; the longitudinal slot being capable of accepting an intramedullary nail and allowing it to pass through the lag screw;
**characterized in that** the dynamic lag screw structure further comprises a longitudinal sheath (103), the longitudinal sheath comprising an upper orifice (109) and a lower orifice (110), the upper orifice and lower orifice together being capable of accepting the intramedullary nail and allowing the intramedullary nail to be passed through the longitudinal sheath.

2. An orthopaedic fixation apparatus and system according to claim 1 and further comprising:
the intramedullary nail (101);
and securing means.

3. The orthopaedic fixation apparatus and system of Claim 2, the intramedullary nail having one or more lateral orifices (105) passing through it; and
the securing means comprising one or more surgical screws (106).

4. The orthopaedic fixation apparatus and system of any one of claims 1 to 3, in which the longitudinal sheath is tapered at its distal end (116).

5. The orthopaedic fixation apparatus and system of any one of claims 1 to 3, in which the longitudinal sheath at its proximal end (117) has an outer rim (118).

6. The orthopaedic fixation apparatus and system any one of claims 1 to 3, in which the longitudinal sheath at its proximal end an inner lip (119).

7. The orthopaedic fixation apparatus and system of any one of claims 1 to 3, in which the lag screw at its distal end has a self-tapping thread (111).

8. The orthopaedic fixation apparatus and system of any one of claims 1 to 3, in which the lag screw has a drive (112) with internal threading (113) capable of receiving a self-holding screwdriver.

9. The orthopaedic fixation apparatus and system of any one of claims 1 to 3, in which the lag screw has at its proximal end an external lip (120) capable of engaging an inner lip of a longitudinal sheath.

10. The orthopaedic fixation apparatus and system of any one of claims 1 to 3, in which the intramedullary nail, at its proximal end (108), is configured to receive a guidance device.

11. The orthopaedic fixation apparatus and system of Claim 3,
wherein the intramedullary nail at its proximal end, is configured to receive a guidance device;
wherein the longitudinal sheath is tapered at its distal end and comprises an external rim and an inner lip at its proximal end ; and
wherein the lag screw employs at its distal end a self-tapping thread, and at its proximal end a drive with internal threading capable of receiving a self-holding screwdriver or comparable instrument and an eternal lip capable of engaging an inner lip of a longitudinal sheath.

## Patentansprüche

1. Orthopädische(s) Fixierungsvorrichtung und -system, umfassend:
eine dynamische Zugschraubenstruktur (102), die eine Zugschraube (104) umfasst, wobei die Zugschraube einen Längsschlitz (114) durch einen Schaft (115) der Zugschraube umfasst;
wobei der Längsschlitz in der Lage ist, einen Marknagel aufzunehmen und ihn durch die Zugschraube hindurchgehen zu lassen;
**dadurch gekennzeichnet, dass** die dynamische Zugschraubenstruktur ferner einen Längshülse (103) umfasst, wobei die Längshülse eine obere Öffnung (109) und eine untere Öffnung (110) umfasst, wobei die obere Öffnung und die untere Öffnung gemeinsam geeignet sind, den Marknagel aufzunehmen und diesem zu ermöglichen, durch die Längshülse hindurch geführt zu werden.

2. Orthopädische(s) Fixierungsvorrichtung und -system nach Anspruch 1 und ferner umfassend:
den Marknagel (101);
und Sicherungsmittel

3. Orthopädische(s) Fixierungsvorrichtung und -system nach Anspruch 2, wobei der Marknagel eine oder mehrere durch diesen verlaufende seitliche Öffnungen (105) aufweist;
und wobei das Sicherungsmittel eine oder mehrere chirurgische Schraube(n) (106) umfasst.

4. Orthopädische(s) Fixierungsvorrichtung und -system nach einem der Ansprüche 1 bis 3, bei denen sich die Längshülse an ihrem distalen Ende (116) verjüngt.

5. Orthopädische(s) Fixierungsvorrichtung und -system nach einem der Ansprüche 1 bis 3, bei denen die Längshülse an ihrem proximalen Ende (117) einen äußeren Rand (118) aufweist.

6. Orthopädische(s) Fixierungsvorrichtung und -system nach einem der Ansprüche 1 bis 3, bei denen die Längshülle an ihrem proximalen Ende eine innere Lippe (119) aufweist.

7. Orthopädische(s) Fixierungsvorrichtung und -system nach einem der Ansprüche 1 bis 3, bei denen die Zugschraube an ihrem distalen Ende ein selbstschneidendes Gewinde (111) aufweist.

8. Orthopädische(s) Fixierungsvorrichtung und -system nach einem der Ansprüche 1 bis 3, bei denen die Zugschraube einen Antrieb (112) mit Innengewinde (113) aufweist, der geeignet ist, einen selbsthaltenden Schraubendreher aufzunehmen.

9. Orthopädische(s) Fixierungsvorrichtung und -system nach einem der Ansprüche 1 bis 3, bei denen die Zugschraube an ihrem proximalen Ende eine äußere Lippe (120) aufweist, die geeignet ist, in eine innere Lippe einer Längshülse einzugreifen.

10. Orthopädische(s) Fixierungsvorrichtung und -system nach einem der Ansprüche 1 bis 3, bei denen der Marknagel an seinem proximalen Ende (108) dafür konfiguriert ist, eine Führungsvorrichtung aufzunehmen.

11. Orthopädische(s) Fixierungsvorrichtung und -system nach Anspruch 3, wobei der Marknagel an seinem proximalen Ende dafür konfiguriert ist, eine Führungsvorrichtung aufzunehmen;
wobei die Längshülse an ihrem distalen Ende verjüngt ist und an ihrem proximalen Ende einen äußeren Rand und eine innere Lippe umfasst; und
wobei die Zugschraube an ihrem distalen Ende ein selbstschneidendes Gewinde und an ihrem proximalen Ende einen Antrieb mit Innengewinde, der einen selbsthaltenden Schraubendreher oder ein vergleichbares Instrument aufzunehmen vermag, und eine äußere Lippe verwendet, die geeignet ist, in eine innere Lippe einer Längshülse einzugreifen.

## Revendications

1. Appareil et système de fixation orthopédique comprenant :
une structure de vis tire-fond dynamique (102) comprenant une vis tire-fond (104), la vis tire-fond comprenant une fente longitudinale (114) à travers une tige (115) de la vis tire-fond ;
la fente longitudinale pouvant accepter un clou intramédullaire et lui permettre de passer à travers la vis tire-fond ;
**caractérisé en ce que** la structure de vis tire-fond dynamique comprend en outre une gaine longitudinale (103), la gaine longitudinale comprenant un orifice supérieur (109) et un orifice inférieur (110), l'orifice supérieur et l'orifice inférieur étant ensemble capables d'accepter le clou intramédullaire et permettant au clou intramédullaire de passer à travers la gaine longitudinale.

2. Appareil et système de fixation orthopédique selon la revendication 1 et comprenant en outre :
le clou intramédullaire (101) ;
et des moyens de fixation.

3. Appareil et système de fixation orthopédique selon la revendication 2, le clou intramédullaire comportant un ou plusieurs orifices latéraux (105) traversant celui-ci ;
et les moyens de fixation comprenant une ou plusieurs vis chirurgicales (106).

4. Appareil et système de fixation orthopédique selon l'une quelconque des revendications 1 à 3, dans lequel la gaine longitudinale est effilée à son extrémité distale (116).

5. Appareil et système de fixation orthopédique selon l'une quelconque des revendications 1 à 3, dans lequel la gaine longitudinale à son extrémité proximale (117) comporte un rebord extérieur (118).

6. Appareil et système de fixation orthopédique selon l'une quelconque des revendications 1 à 3, dans lequel la gaine longitudinale à son extrémité proximale comporte une lèvre intérieure (119).

7. Appareil et système de fixation orthopédique selon l'une quelconque des revendications 1 à 3, dans lequel la vis tire-fond à son extrémité distale comporte un filetage autotaraudeur (111).

8. Appareil et système de fixation orthopédique selon l'une quelconque des revendications 1 à 3, dans lequel la vis tire-fond est dotée d'un entraînement (112) comportant un filetage intérieur (113) capable de recevoir un tournevis à auto-maintien.

9. Appareil et système de fixation orthopédique selon l'une quelconque des revendications 1 à 3, dans lequel la vis tire-fond comporte à son extrémité proximale une lèvre extérieure (120) capable de venir en prise avec une lèvre intérieure d'une gaine longitudinale.

10. Appareil et système de fixation orthopédique selon l'une quelconque des revendications 1 à 3, dans lequel le clou intramédullaire, à son extrémité proximale (108), est configuré pour recevoir un dispositif de guidage.

11. Appareil et système de fixation orthopédique selon la revendication 3, dans lequel le clou intramédullaire, à son extrémité proximale, est configuré pour recevoir un dispositif de guidage ;
dans lequel la gaine longitudinale est effilée à son extrémité distale et comprend un rebord extérieur et une lèvre intérieure à son extrémité proximale ; et
dans lequel la vis tire-fond emploie, à son extrémité distale, un filetage autotaraudeur, et à son extrémité proximale, un entraînement à filetage intérieur capable de recevoir un tournevis à auto-maintien ou un instrument comparable et une lèvre extérieure capable de venir en prise avec une lèvre intérieure d'une gaine longitudinale.
